# FASCICULE DE BREVET EUROPEEN

(11) **EP 2 359 220 B1**
(45) Date de publication et mention de la délivrance du brevet: **24.06.2020**
(21) Numéro de dépôt: 09756534.5
(22) Date de dépôt: 19.10.2009
(51) Int. Cl.: G06F 3/01, H04N 5/235, H04N 13/00, A61B 3/113, G03B 15/16, G06K 11/00, G06K 9/52, G05B 19/00, G06F 3/033

(54) **DISPOSITIF ET PROCÉDÉ DE SYNCHRONISATION D'UNE ONDE LUMINEUSE AVEC UN SIGNAL ELECTRONIQUE**
EINRICHTUNG UND VERFAHREN ZUM SYNCHRONISIEREN EINER LICHTWELLE MIT EINEM ELEKTRONISCHEM SIGNAL
DEVICE AND METHOD FOR SYNCHRONISING A LIGHT WAVE WITH AN ELECTRONIC SIGNAL

(30) Priorité: 21.10.2008 FR 0805830
(43) Date de publication de la demande: 24.08.2011
(73) Titulaire: Guitteaud, Eric, 75020 Paris (FR); Bascoulergue, Pierre, 75020 Paris (FR)
(72) Inventeur: Guitteaud, Eric, 75020 Paris (FR); Bascoulergue, Pierre, 75020 Paris (FR)
(74) Mandataire: Fédit-Loriot
(86) Numéro de dépôt international: PCT/FR2009/001220
(87) Numéro de publication internationale: WO 2010/046557

(56) Documents cités:
- WO-A2-2004/084054
- US-A- 4 836 670
- US-A1- 2002 013 573
- US-A1- 2005 175 218

## Description

L'invention se rapporte à un procédé de synchronisation d'une onde lumineuse avec un signal électronique, et à un dispositif de mise en œuvre du procédé.

### DOMAINE TECHNIQUE DE L'INVENTION

La présente invention se rapporte au domaine des dispositifs de suivi de mouvements à partir de l'identification et la détermination de la position d'une onde lumineuse ou de son reflet sur un objet, capté par un appareil de prise de vue, tels que dans des dispositifs de suivi de mouvements de l'axe optique d'un œil.

### ETAT DE LA TECHNIQUE ANTERIEURE

Dans de nombreux domaines, il est intéressant d'utiliser des applications industrielles dans lesquelles il est avantageux de disposer de signaux électroniques, tels que des signaux vidéos, proportionnels aux variations d'orientation de l'axe optique de l'œil.

Ces applications industrielles comprennent l'utilisation de l'œil pour la commande de systèmes. Par exemple pour contrôler un ordinateur, un téléviseur, un écran de cinéma, un cockpit d'avion d'un simple mouvement de l'œil.

L'avantage de dispositifs de suivi des mouvements de l'œil est de permettre d'identifier l'objet du regard d'un sujet dans une scène présente devant lui.

Dans l'art antérieur, on distingue deux grandes familles de dispositifs de suivi des mouvements de l'œil:
- les dispositifs non intrusifs et non portables ;
- les dispositifs intrusifs et portables.

Tous ces dispositifs utilisent un principe technique de base de suivi du mouvement des yeux : l'identification de la position de l'œil par rapport à un point lumineux réfléchi sur la cornée nommé « point de Purkinje ».

Le principe consiste à disposer une DEL (diode électroluminescente) émettant un rayonnement dans les longueurs d'ondes de l'infrarouge juste à côté de l'objectif de la caméra qui prend des images de l'œil. Cette DEL infrarouge émet une lumière vers l'œil dans la même direction que l'axe de l'objectif de la caméra vidéo. Cette lumière se réfléchit sur la cornée de l'œil : un point lumineux apparaît alors sur l'image de la cornée de l'œil capturée par la caméra.

Ce dispositif non-intrusif est un dispositif qui permet d'enregistrer les mouvements de l'œil donc de suivre le regard lorsque le sujet regarde par exemple un écran d'ordinateur.

Ce type de système est généralement composé d'une caméra très haute définition disposée à une soixantaine de centimètre ou plus de la tête du sujet dans l'axe du regard du sujet.

Cette caméra étant de haute définition, il est possible alors de reconnaître le visage du sujet auquel elle fait face. Puis par traitement d'image et reconnaissance de formes, il est possible de reconnaître l'emplacement des yeux et de faire un agrandissement dans l'image pour déterminer avec précision la position de la pupille par rapport au point de Purkinje. Ce dispositif n'interagit pas avec le sujet. Le sujet n'a pas à porter de lunette spéciale ou de casque. De plus il ne nécessite pas de mise au point mécanico-optique à réaliser pour avoir une image nette de l'œil. Le dispositif peut être automatisé et motorisé.

Toutefois un tel dispositif a pour principal inconvénient, de reposer sur l'utilisation d'une caméra haute définition dont le coût dépasse de plus de 100 fois le prix d'une caméra à résolution standard. De plus, lors de l'analyse du regard, le sujet ne peut pas bouger la tête au-delà d'une limite tridimensionnelle définie par l'angle de vue de la caméra qui filme l'œil.

Les dispositifs intrusifs permettent d'enregistrer ce suivi du regard lorsque l'individu regarde une scène globale comme le cockpit d'un avion par exemple.

Ce type de dispositif est généralement composé d'une caméra disposée à une dizaine de centimètre de la tête du sujet dans l'axe de regard du sujet. Cette caméra est proche de la tête, elle doit donc être fixée à une paire de lunette spécifique ou à un casque.

Cette caméra filmant directement la zone de l'œil, par traitement d'image il est possible de reconnaître avec précision la position de la pupille par rapport au point de Purkinje.

Ainsi, ce dispositif confère à l'individu une grande liberté de mouvement. Il peut ainsi se déplacer tout en analysant la direction de son regard.

De plus, un tel dispositif permet l'utilisation de tous types de caméras ayant un coût réduit comparées par exemple aux caméras à haute définition.

Cependant, un inconvénient de ce dispositif se rapporte au fait que l'individu doit porter une paire de lunettes spéciale ou encore un casque approprié sur lequel est disposé la caméra et la DEL infrarouge.

De plus, un tel dispositif nécessite que soit réalisée une mise au point mécanico-optique pour avoir une image nette de l'œil en fonction du sujet. Le système ne peut pas être automatisé et motorisé car le système doit rester petit et léger puisqu'il sera porté par le sujet.

Un problème récurrent se pose alors de reconnaître la position du centre de la pupille par rapport au point de Purkinje dans l'image de l'œil capturée par la caméra, quel que soit le dispositif de détection de mouvements utilisé.

En effet, en fonction de l'environnement lumineux dans lequel le sujet se trouve, il est possible que des artéfacts rendent difficile la reconnaissance du point de Purkinje par simple traitement d'image.

Pour pallier ce problème, on connaît dans l'art antérieur des dispositifs de traitement de l'image coûteux et consommateurs en temps de calcul et des modèles probabilistes qui identifient au mieux l'emplacement du point de Purkinje.

En effet, afin de simplifier l'identification du point de Purkinje, les dispositifs actuels de détection de mouvements de l'œil utilisent un principe de synchronisation de l'allumage de la DEL de réflexion du point de Purkinje, dit DEL de Purkinje, avec la capture de l'image de l'œil. Ainsi, la DEL de Purkinje est allumée une image sur deux.

Alors, si on effectue la différence de la valeur de la luminosité de chaque point de la séquence de l'image avec la DEL de Purkinje allumée et de l'image avec la DEL de Purkinje éteinte, on obtient une troisième image résultante qui fait apparaître très clairement le point de Purkinje même dans un environnement bruité.

Après traitement de l'image différentielle, on obtient la position du point de Purkinje sans être gêné par les artefacts ambiants.

Pour synchroniser l'allumage de la DEL de Purkinje avec la capture d'une image il est nécessaire de contrôler le déclenchement de la prise des images par la caméra.

Pour ce faire, la camera doit rendre disponible un connecteur électrique qui permet de fournir à la caméra un top synchronisation. Les caméras avec entrée de synchronisation externe possèdent la faculté de synchroniser la cadence de prise d'image à l'aide d'une horloge extérieure à la caméra. Ainsi, l'horloge extérieure commande en même temps l'allumage ou l'extinction de la DEL et la prise d'image de la scène qui comporte la DEL.

Mais ce type d'entrée n'existe que sur certains modèles de caméras. Pour les autres modèles, il est nécessaire de démonter la caméra et de trouver un élément du circuit électrique interne à la caméra qui pourrait faire office d'entrée de synchronisation externe. Ce chantier est trop complexe et totalement dépendant du modèle de caméra utilisée.

Ainsi, un des inconvénients majeurs de tels dispositifs est de ne pas être compatible et portable sur n'importe quel type d'appareil de prise de vue.
Parmi les dispositifs de suivi des mouvements de l'œil connus de l'art antérieur, on peut citer ceux des documents WO2004/084054, US2005/175218, US 4836670 et US2002/013573. En particulier, le document WO2004/084054 fait connaître un système de suivi de la direction du regard basé sur la détection, à l'aide d'une caméra, de la réflexion sur l'œil d'une onde lumineuse émise vers l'œil. Ce document décrit de réaliser une synchronisation de l'allumage d'une source lumineuse permettant de déterminer un point de Purkinie en commandant l'allumage de la source lumineuse alternativement une image sur deux, sur la durée d'une image entière.

### EXPOSE DE L'INVENTION

L'invention vise à remédier aux inconvénients de l'état de la technique et en particulier aux problèmes liés à la difficulté technique rencontrée pour la synchronisation avec une horloge extérieure d'appareils de prise de vue dépourvus de connecteur électrique permettant de fournir audit appareil un top synchronisation extérieur.

L'invention propose de réaliser une telle synchronisation avec tous les types d'appareils analogiques ou numériques de prise de vue en mode entrelacé par l'utilisation de l'horloge interne de ces appareils afin de réaliser la synchronisation de l'allumage de la DEL de Purkinje.

Plus précisément, l'invention a pour objet un procédé selon la revendication 1 et un système selon la revendication 3.

### BREVE DESCRIPTION DES FIGURES

D'autres caractéristiques et avantages de l'invention ressortiront à la lecture de la description qui suit, en référence aux figures annexées, qui illustrent à:
la figure 1A, une vue schématique d'un sujet vue de face ;
la figure 1B, une vue schématique d'un sujet vue de profil ;
la figure 1C, le boîtier de détection du signal de synchronisation selon un mode de réalisation de l'invention ;
la figure 2, une représentation détaillée d'une ligne vidéo analogique selon l'état de la technique ;

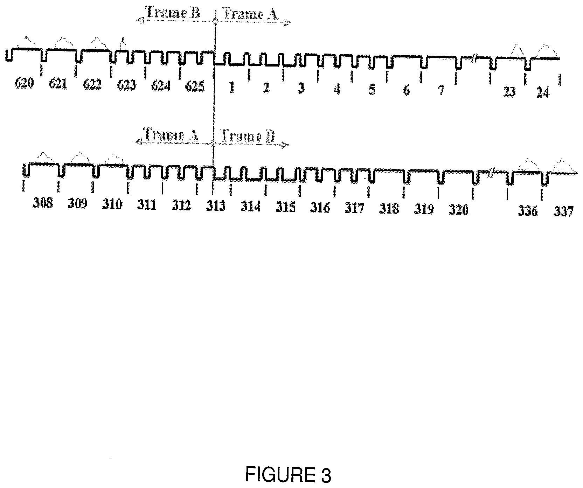

la figure 3, une représentation détaillée de synchro trame analogique selon l'état de la technique ;

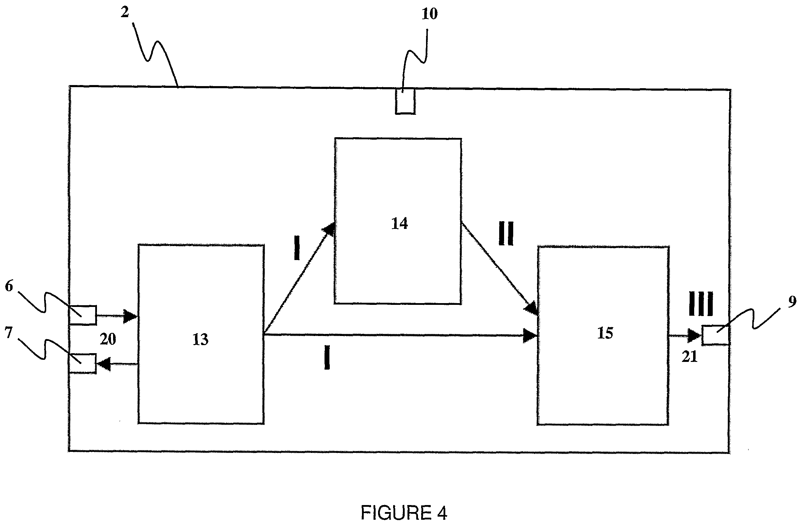

la figure 4, une représentation schématique du synoptique fonctionnel selon l'invention ;
la figure 5A, une représentation de graphes électriques relatifs au traitement du signal électronique à la sortie de l'appareil de prise de vue, selon l'invention ;
la figure 5B, une représentation d'un graphe électrique relatif au comportement de l'allumage de la source lumineuse en fonction de la capture des images successives par l'appareil de prise de vue;
- la figure 6, un schéma électrique du circuit compris dans le boîtier, reproduisant le synoptique, selon l'invention, et
- la figure 7, un autre mode de réalisation du schéma électrique du circuit compris dans le boîtier, selon l'invention ;
- La figure 8, une représentation de la succession de deux images contenant une source lumineuse commandée par le boîtier de détection du signal de synchronisation capturées par l'appareil de prise de vue et l'image résultante de la différence des niveau de luminosité des deux images.

Pour plus de clarté, les éléments identiques ou similaires sont repérés par des signes de référence identiques sur l'ensemble des figures.

### DESCRIPTION DETAILLEE D'UN MODE DE REALISATION

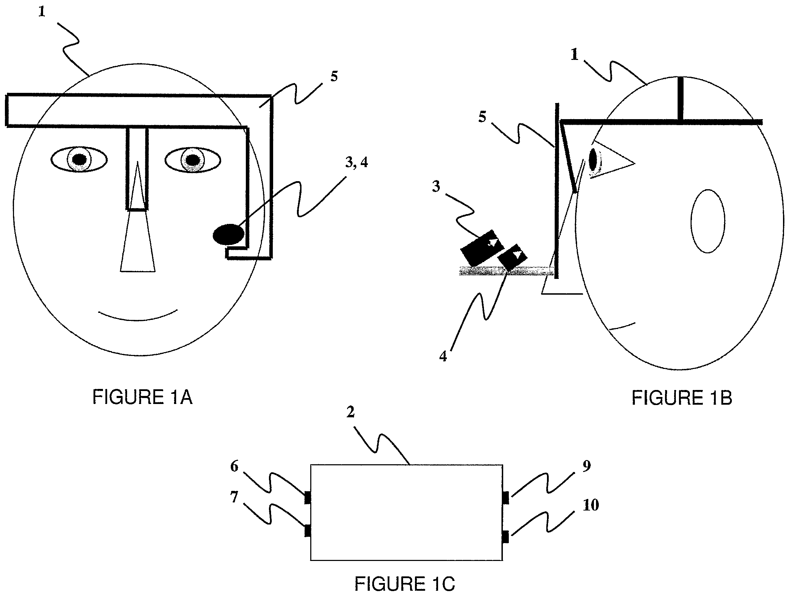

Dans un exemple de réalisation du dispositif, selon l'invention, illustré à la figure 1A et 1B, ce dispositif comprend :
- un élément 5 apte à être placé sur la tête du sujet 1 ;
- un appareil 3 de prise de vue ;
- une source lumineuse 4, tel qu'une diode 4 ;
- un moyen de stockage, et
- un boîtier 2 de détection du signal de synchronisation.

L'élément 5 correspond à un casque sur lequel est fixé l'appareil de prise de vue 3 et la diode 4.

Cet élément 5 est placé sur la tête 1 du sujet pour lequel on souhaite identifier la position de l'œil.

L'élément 5 peut se rapporter aussi à une paire de lunettes spécifique.

L'appareil 3 de prise de vue correspond à une caméra vidéo.

La diode 4 comporte deux pôles, anode et cathode entre lesquelles le courant ne peut passer que dans un sens : de l'anode vers la cathode, et émet une énergie lumineuse sous l'influence de l'énergie électrique correspondant à un rayonnement monochromatique incohérent, sans que celle-ci soit transformée en chaleur. La diode 4 utilisée dans ce dispositif est dite électroluminescente (abréviation de DEL en français, et LED en anglais) et émet un rayonnement lumineux émis est compris dans les longueurs d'ondes correspondant à l'infrarouge.

Cette diode électroluminescente (DEL) 4 est associée à la caméra 3 de sorte à déterminer le point de Purkinje.

Plus particulièrement, cette DEL 4 est une DEL de Purkinje qui est disposée à proximité du centre de l'objectif de cette caméra 3.

La caméra 3 est connectée directement au boîtier 2 de détection du signal de synchronisation contenu dans le signal électronique et de commande de l'allumage de la DEL de Purkinje 4.

Dans ce mode de réalisation, le signal électronique se rapporte à un signal vidéo qui est soit analogique, ou soit numérique. Ce signal véhicule une succession d'images comportant des trames paires et impaires entrelacées.

Ce boîtier 2 comprend une interface d'entrée 6 et de sortie 7, un module 9 d'alimentation de DEL et un module d'alimentation du boîtier 10.

Dans ce mode de réalisation de l'invention, les interfaces 6 et 7 sont des interfaces vidéo.

Le boîtier 2 peut contenir des modules sans fil permettant de recevoir et transmettre le flux vidéo et des signaux permettant de commander à distance l'alimentation de la DEL.

Le boîtier selon l'invention comprend des moyens de traitement permettant d'identifier et d'analyser les images et trames reçues de la caméra 43.

Ces moyens de traitement correspondent par exemple à un microprocesseur associé à des moyens de mémoire volatiles et/ou non volatiles.

Le boîtier comprend aussi des moyens de connexion 7, correspondant à l'interface de sortie 7, compatible avec un moyen de stockage.

Ce moyen de stockage est apte à enregistrer les images comprises dans le signal vidéo.

Ce moyen de stockage correspond :
- aux moyens de mémoire du boîtier ;
- à des moyens de mémoire amovible tel qu'une clef USB ou encore un disque dur portable ;
- à une carte mémoire amovible de stockage, compris dans des équipements électroniques ;
- à un disque optique de type CD (acronyme de « compact Disc » qui signifie en français disque compact), DVD (acronyme de « Digital Video Disc »qui signifie en français disque vidéo numérique), HD-DVD (acronyme de « High Density Digital Versatile Disc » qui signifie en français disque numérique polyvalent de haute densité), BRD (acronyme de « Blue-Ray Disc » qui signifie en français disque Blue-Ray) ; et donc
- à tous supports comprenant des moyens de mémoire flash, volatile, non volatile, mémoire morte.

Les moyens de connexion 7 de ce boîtier 2 correspondent, de manière non limitative, à :
- un lecteur/graveur de disque optique,
- un bus informatique PNP (acronyme de « plug-and-play » qui est une procédure permettant aux périphériques récents d'être reconnus rapidement et automatiquement par le système d'exploitation) tel que
   - une prise USB;
   - une prise eSATA;
   - une prise Firewire;
- un module lecture/écriture multi carte mémoire ;
- un module IrDA (Infrared Data Association) qui permet d'échanger des données par ondes infrarouges avec un autre périphérique équipé de cette technologie ;
- un module d'échange des données par liaison radio tel qu'un module :
   - Wi-Fi (abbreviation de wireless fidelity), et
   - Bluetooth.

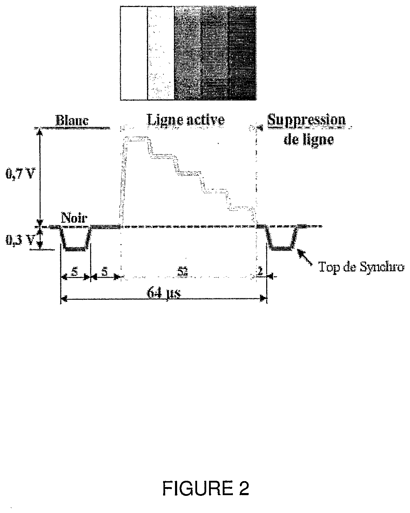

À la figure 2, est représenté un exemple de signal vidéo monochrome d'une caméra analogique de type PAL- 50Hz pour une ligne vidéo monochrome, ce signal est composé de deux parties :
- une information vidéo, d'amplitude de 0,7 V avec la couleur noir qui représente le niveau d'énergie le plus bas, et donc la tension la plus basse, et
- une information de synchronisation, matérialisée par une impulsion négative de 0,3 V.

Cette ligne vidéo correspond aussi au détail d'une ligne d'une image de télévision comprenant 625 lignes avec un cadencement de trames à 50Hz, et une durée de ligne de 64µs.

Le top de synchro de trame représente le temps pendant lequel le signal est constant (palier à la figure 2).

La récupération de la synchronisation de trame exploite l'inversion du top de synchro pendant 2,5 lignes : l'impulsion négative devient positive, la valeur moyenne décroit, il suffit alors d'un filtre passe-bas pour extraire le top de synchro trame.

Ainsi, un flux vidéo est composé d'une succession d'images, 25 par seconde en Europe, composant l'illusion du mouvement. Chaque image est décomposée en lignes horizontales, chaque ligne pouvant être considérée comme une succession de points. La lecture et la restitution d'une image s'effectue donc séquentiellement ligne par ligne comme un texte écrit : de gauche à droite puis de haut en bas.

Donc l'image d'un téléviseur est une succession de balayages linéaires horizontaux, de gauche à droite, partant du haut, et finissant en bas de l'écran. Au commencement de la télévision, la qualité des éléments phosphorescents du tube est fort médiocre. De ce fait, quand le faisceau balaye le bas de l'écran, le haut a déjà disparu, d'où un phénomène de scintillement, ressenti fortement par l'œil humain pour 25 Hz.

La solution la plus simple eût été d'accélérer la cadence de balayage, mais ceci imposait également d'augmenter la cadence des images, ce qui était inutile d'un point de vue cinématographique (le mouvement est perçu de la même façon), et fort coûteux en matériel et en bande passante.

Une solution plus astucieuse fut de doubler la cadence de balayage, en omettant une ligne sur deux, afin de garder une quantité d'information constante. Ainsi, une première passe affiche toutes les lignes impaires en deux fois moins de temps que pour une image entière et une seconde passe affiche les lignes manquantes paires : c'est ce que l'on appelle l'entrelacement. On obtient bien le même nombre de lignes de balayages pour une image, et on balaye deux fois l'écran pour afficher une seule image. On désigne par le terme « trame » ("field" en anglais) une passe de balayage.

Une image est donc constituée de deux trames, puisqu'il faut deux balayages pour définir l'image (« frame » en anglais).

La caméra 3, qui fonctionne comme un « téléviseur inversé », adopte elle aussi cet entrelacement du balayage. Dans la première moitié du temps d'une image, une première prise de vue définit toutes les lignes impaires, et une moitié d'image plus tard, une seconde prise de vue définit les lignes paires. Les deux prises de vues sont distantes dans le temps, de sensiblement une moitié d'image.

Le boîtier 2 selon l'invention utilise des composants électroniques afin d'extraire du signal vidéo de la caméra 3 le top trame de synchronisation du signal vidéo et de le traiter pour obtenir l'information de top image paire et top image impaire.

Rappelons qu'une image est composée de deux trames (une trame impaire et une trame paire) et qu'un flux vidéo est composé d'une succession d'images numérotées de 1 à l'infini dont le groupe des images impaires (numérotées 1, 3, 5, ...) et le groupe des images paires (numérotées 2, 4, 6, ...). Donc, quand deux images se succèdent l'une est impaire et l'autre est forcément paire.

Comme nous l'avons vue précédemment les notions de trame impaire/paire et d'image impaire/paire sont différentes.

Pour déterminer la direction du regard de l'œil du sujet, la caméra 3 échange des signaux avec le boîtier 2 qui active ou pas la DEL 4 de Purkinje.

Lorsque la caméra 3 capture des images de l'œil du sujet celles-ci sont : transmises au boîtier 2 sous forme d'un signal électronique, le signal vidéo, à partir d'une liaison qui est connectée à une de ses extrémités à l'interface d'entrée 6 vidéo du boîtier 2 et transmises au moyen de stockage par le biais de moyens de connexion correspondant à l'interface de sortie vidéo 7.

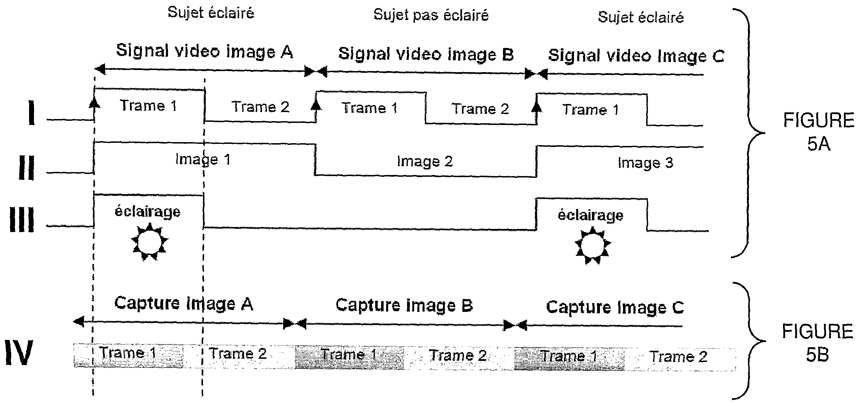

Le traitement du signal vidéo provenant de la sortie vidéo de la caméra 3 est représenté à la figure 5A. Ce signal correspond aux images A, B, C, lorsque le sujet est éclairé ou non éclairé.

Ce traitement correspond à la détection du top de synchronisation de la trame impaire de l'image impaire afin d'allumer la DEL pendant le même intervalle de temps que la trame impaire et d'éteindre la DEL pendant tout le reste du temps.

L'information de synchronisation de trame comprise dans ce signal fait l'objet d'un traitement par les moyens de traitement du boîtier 2 lors d'une étape 13 de détection du top de trame impaire. Cette étape 13 permet ainsi de repérer et d'extraire les trames impaires (état électrique haut) et paires (état électrique bas) restitués par le signal vidéo, au point I.

À l'étape 14 de détection du top image, les images paires et impaires restituées par le signal vidéo sont repérées et les images impaires sont identifiées par un état électrique haut, au point II.

Les signaux obtenus aux étapes 13 et 14 sont utilisés lors d'une étape 15 afin d'effectuer une commande de déclenchement/d'extinction de la DEL 4 de Purkinje à partir d'un calcul effectué par les moyens de traitement du boîtier en faisant un ET logique entre les signaux des points I et II, identifiés par un état électrique haut des trames impaires et des images impaires restituées par le signal vidéo.

L'étape 15 permet la transmission de la commande à la DEL 4.

Parallèlement au traitement des informations de trames prélevées et traitées à partir de ce signal vidéo lors des étapes 13, 14, 15, ce signal video est retransmis à un enregistreur vidéo sans modification et tel qu'il a été reçu sur l'entrée 6 vers la sortie 7 vidéo à laquelle est connecté l'enregistreur.

Lorsque la DEL 4 de Purkinje est allumée au moment où est identifiée la trame impaire de l'image impaire dans le signal vidéo, cela équivaut à éclairer la scène de la trame impaire que la caméra 3 est en train de capturer.

De plus, de par la conception du montage électrique qui repère la durée d'une trame, on voit que la durée d'éclairage (entre les deux lignes verticales en pointillés) est rigoureusement égale au temps d'une seule trame (soit le temps d'une demi-image).

Si, pour des raisons techniques propres à la caméra 3, l'allumage de la DEL 4 de Purkinje démarre un peu après le début de la capture de l'image entière, on est quand même sûr et certain que l'éclairage se fera obligatoirement pendant une image complète et ne pourra pas se chevaucher sur l'image suivante.

En revanche, l'éclairage risque de se faire à cheval sur la fin de la trame impaire et le début de la trame paire. Ce problème n'est pas bloquant car par un traitement approprié de l'image résultante (somme de l'entrelacement des deux trames), il possible par interpolation de reconstituer une image uniformément éclairée.

L'allumage de la DEL 4 de Purkinje s'effectuant sur la durée d'une trame seulement et démarrant au début d'une image complète, on est certain que le système fonctionne quel que soit le type du signal vidéo même si il y a un décalage entre le top image reçu dans le signal vidéo et le top de la capture réelle de l'image de la caméra.

Ce décalage sera forcément compris entre 0 ms (pas de décalage) et le temps de restitution d'une trame est par exemple de 20 ms pour un signal analogique PAL-50Hz (voir graphe IV figure 5B).

Ainsi, la synchronisation de la capture ne nécessite pas d'étalonnage préliminaire en fonction du modèle de caméra utilisé.

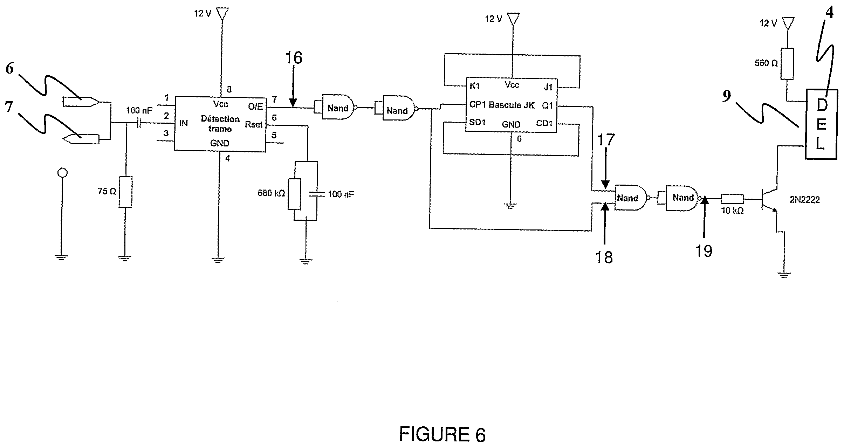

À la figure 6, est représenté un schéma électrique du système compris dans le boîtier dans le cas du traitement d'un signal vidéo analogique PAL, SECAM ou NTSC.

Le signal vidéo provenant de la caméra (3) entre dans le boîtier par le connecteur (6). Le circuit de détection des tops trame (comme le circuit LM1881) permet l'extraction du top synchronisation des trames paires et impaires : le point de mesure (16) correspond à la courbe I de la figure 5A. La bascule JK (type CMOS 4027) détecte le top synchronisation des images paires et impaires : le point de mesure (17) correspond à la courbe II de la figure 5A. Les deux portes NAND (type deux quart de circuit CMOS 4011) ne change pas le signal du top synchronisation des trames paires et impaires : le point de mesure (18) correspond à la courbe de la figure 5A. Les deux portes NAND (type deux quart de circuit CMOS 4011) constitue la logique combinatoire ET pour restituer le signal d'allumage à la DEL : le point de mesure (19) correspond à la courbe III de la figure 5A.

Par ailleurs, dans un autre mode de réalisation, une logique qui génère l'allumage de la DEL 4 de Purkinje pendant un temps strictement égal à une trame, par exemple 20 ms, peut être remplacée une logique de temporisateur (ou tout autre système de temporisation) qui à chaque top de trame impaire d'une image impaire déclenche un monostable qui allume la DEL 4 de Purkinje pendant un temps sensiblement égal à celui d'une trame.

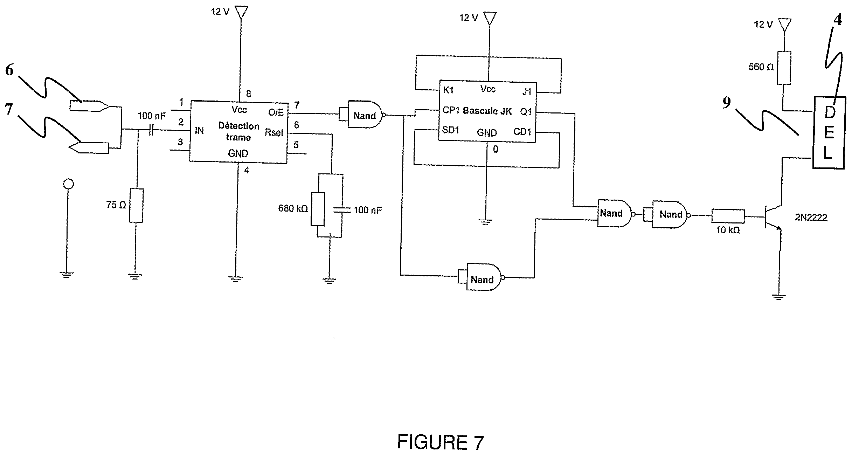

Par ailleurs, une variante du montage électronique est illustrée à la figure 7. Ce montage comprend une modification qui le rend différent du montage de la figure 6.

En effet, elle diffère du montage de la figure 6 par le déplacement d'une porte NAND à l'entrée de la bascule JK.

Cette modification a pour effet d'allumer la DEL 4 pendant les trames impaires des images paires.

Le signal vidéo provenant de la camera 3 est retransmis à un enregistreur vidéo par le connecteur 7. En temps réel, ou bien en temps décalé, il est alors possible de récupérer la numérisation des images successives capturées par la caméra 3.

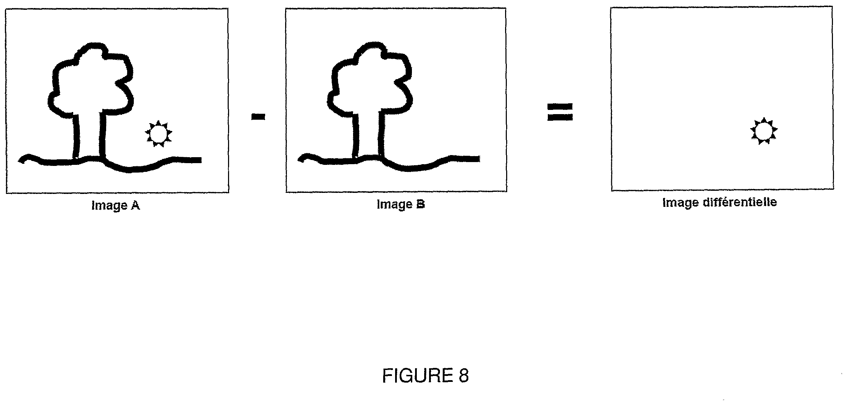

Ainsi, pour deux images successives capturées, grâce au procédé de l'invention, une des deux images contient l'image de la DEL allumée (image A dans la figure 8) et l'autre image non (image B dans la figure 8).

En effectuant une soustraction point à point de la valeur de la luminosité entre les deux images avec seuillage, on obtient une image résultante qui contient uniquement l'image de la DEL allumée.

A partir d'algorithmes particuliers, la position de la DEL est alors déterminée dans l'image A ou B.

Dans le cas du suivi du mouvement des yeux, cette position de la réflexion de la DEL 4 sur l'œil correspond à la position du point de Purkinje.

Ainsi à l'aide de la position du centre de la pupille de l'œil et à la position du point de Purkinje, il est possible de déterminer l'angle de regard d'un œil par rapport à une scène.

L'invention n'est pas limitée aux exemples de réalisation décrits et illustrés. Elle n'est en outre pas limitée à ces exemples d'exécution et aux variantes décrites, notamment en utilisant d'autres types de circuits électroniques que le LM1881, CMOS 4027 ou CMOS 4011. Le principe de ces différents circuits peut être réalisé aussi intégralement à l'aide de transistors, transistors à effet de champ, comparateurs analogiques, résistances et condensateurs associés mais aussi à l'aide de circuits hybrides.

## Revendications

1. Procédé de suivi de l'axe optique d'un œil, comprenant la synchronisation d'une onde lumineuse fournie par une source lumineuse (4) avec un signal vidéo capturé par une caméra vidéo (3) comportant des images constituées de trames entrelacées, dans lequel ladite caméra vidéo et ladite source lumineuse sont portées par un élément de support (5) adapté à être ajusté à la tête d'un sujet de manière à capturer des images de l'œil du sujet à l'aide de la caméra vidéo, ladite source lumineuse (4) étant agencée à proximité du centre de l'objectif de la caméra vidéo (3) de sorte à déterminer un point de Purkinje, ledit procédé étant **caractérisé en ce qu'**il comprend les étapes suivantes de:
- réception (20) du signal vidéo par un boîtier (2) de commande de l'allumage de la source lumineuse ;
- extraction (13) par des moyens de traitement du boîtier d'un signal de synchronisation de trame (I) par identification des trames paires et impaires constituant le signal vidéo;
- détermination (14) par les moyens de traitement du boîtier d'un signal de synchronisation des images (II) par détection des images paires et impaires constituant le signal vidéo, la détermination du signal de synchronisation des images paires et impaires constituant le signal vidéo étant obtenue à partir du signal de synchronisation de trame extrait du signal vidéo par les moyens de traitement du boîtier;
- génération (15) d'un signal de commande (III) de la source lumineuse par les moyens de traitement du boîtier à partir d'une combinaison logique entre les signaux de synchronisation de trame (I) et de synchronisation des images (II), propre à provoquer l'allumage de la source lumineuse seulement pendant la durée d'une seule trame et ce, une image sur deux, et
- transmission (21) du signal de commande à un module d'alimentation de la source lumineuse apte à contrôler l'allumage et l'extinction de la source lumineuse en fonction du signal de commande,
et dans lequel le signal vidéo est retransmis par le boîtier à un moyen de stockage pour y être enregistré, ledit signal enregistré faisant l'objet d'un traitement dans lequel on effectue une soustraction point à point de la valeur de luminosité entre deux images successives de sorte à localiser la position dans une scène de la représentation de la source lumineuse.

2. Procédé selon la revendication 1, dans lequel le signal vidéo est analogique ou numérique.

3. Système de suivi de l'axe optique d'un œil, comprenant la synchronisation d'une onde lumineuse avec un signal vidéo comportant des images constituées de trames entrelacées, ledit système comprenant une caméra vidéo (3) apte à fournir le signal vidéo et une source lumineuse (4) apte à fournir l'onde lumineuse ladite caméra vidéo et ladite source lumineuse étant portées par un élément de support (5) adapté à être ajusté à la tête d'un sujet de manière à capturer des images de l'œil du sujet à l'aide de la caméra vidéo, ladite source lumineuse (4) étant agencée à proximité du centre de l'objectif de la caméra vidéo (3) de sorte à déterminer un point de Purkinje, ledit système étant **caractérisé en ce qu'**il comprend un boîtier (2) de commande de l'allumage de la source lumineuse comprenant un module (9) d'alimentation de la source lumineuse (4), une interface d'entrée (6) apte à recevoir le signal vidéo fourni par la caméra vidéo, et des moyens de traitement aptes à extraire du signal vidéo reçu un signal de synchronisation de trame par identification des trames paires et impaires constituant le signal vidéo et à déterminer, à partir du signal de synchronisation de trame extrait du signal vidéo, un signal de synchronisation des images paires et impaires constituant le signal vidéo, lesdits moyens de traitement étant en outre adaptés pour générer un signal de commande à destination du module d'alimentation de la source lumineuse, à partir d'une combinaison logique entre les signaux de synchronisation de trame et de synchronisation des images, propre à provoquer l'allumage de la source lumineuse seulement pendant la durée d'une seule trame et ce, une image sur deux, le boîtier de commande comprenant une interface de sortie correspondant à des moyens de connexion (7) avec un moyen de stockage apte à enregistrer le signal vidéo reçu, ledit signal enregistré étant adapté à faire l'objet d'un traitement dans lequel on effectue une soustraction point à point de la valeur de luminosité entre deux images successives de sorte à localiser la position dans une scène de la représentation de la source lumineuse.

4. Système selon la revendication 3, **caractérisé en ce que** lesdites interfaces d'entrée (6) et de sortie (7) sont analogiques ou numériques.

5. Système selon la revendication 3 ou 4, **caractérisé en ce que** le moyen de stockage est choisi parmi l'un quelconque au moins des éléments suivants: un disque optique, un élément de mémoire amovible, un élément de mémoire interne du boîtier (2).

6. Système selon l'une quelconque des revendications 3 à 5, **caractérisé en ce que** ladite source lumineuse (4) est une diode électroluminescente (4).

7. Système selon la revendication 6, **caractérisé en ce que** ladite diode électroluminescente (4) est apte à émettre un rayonnement sensiblement compris dans des longueurs d'onde de l'infrarouge.

## Patentansprüche

1. Verfahren zum Verfolgen der optischen Achse eines Auges mit der Synchronisation einer Lichtwelle, die durch eine Lichtquelle (4) geliefert wird, mit einem Videosignal, das durch eine Videokamera (3) erfasst wird, das Bilder umfasst, die aus verschachtelten Einzelbildern gebildet sind, wobei die Videokamera und die Lichtquelle durch ein Stützelement (5) getragen sind, das so ausgelegt ist, dass es auf den Kopf eines Probanden eingestellt wird, um Bilder des Auges des Probanden mit Hilfe der Videokamera zu erfassen, wobei die Lichtquelle (4) in der Nähe des Zentrums des Objektivs der Videokamera (3) angeordnet ist, um einen Purkinje-Punkt zu bestimmen, wobei das Verfahren **dadurch gekennzeichnet ist, dass** es die folgenden Schritte umfasst:
- Empfang (20) des Videosignals durch ein Gehäuse (2) zur Steuerung des Einschaltens der Lichtquelle;
- Extraktion (13) eines Einzelbildsynchronisationssignals (I) durch Identifikation der geraden und ungeraden Einzelbilder, die das Videosignal bilden, durch Verarbeitungsmittel des Gehäuses;
- Bestimmung (14) eines Synchronisationssignals (II) der Bilder durch Detektion der geraden und ungeraden Bilder, die das Videosignal bilden, durch die Verarbeitungsmittel des Gehäuses, wobei die Bestimmung des Synchronisationssignals der geraden und ungeraden Bilder, die das Videosignal bilden, aus dem Einzelbildsynchronisationssignal, das aus dem Videosignal durch die Verarbeitungsmittel des Gehäuses extrahiert wird, erhalten wird;
- Erzeugung (15) eines Steuersignals (III) der Lichtquelle durch die Verarbeitungsmittel des Gehäuses aus einer logischen Kombination zwischen den Einzelbildsynchronisationssignalen (I) und den Synchronisationssignalen (II) der Bilder, das dazu geeignet ist, das Einschalten der Lichtquelle nur während der Dauer eines einzigen Einzelbildes, und zwar jedes zweiten Bildes, hervorzurufen, und
- Übertragung (21) des Steuersignals zu einem Modul zur Versorgung der Lichtquelle, das das Einschalten und das Ausschalten der Lichtquelle in Abhängigkeit von dem Steuersignal steuern kann,
und wobei das Videosignal durch das Gehäuse zu einem Speichermittel übertragen wird, damit es dort aufgezeichnet wird, wobei das aufgezeichnete Signal Gegenstand einer Verarbeitung ist, bei der eine punktweise Subtraktion des Helligkeitswerts zwischen zwei aufeinander folgenden Bildern durchgeführt wird, um die Position der Darstellung der Lichtquelle in einer Szene zu lokalisieren.

2. Verfahren nach Anspruch 1, wobei das Videosignal analog oder digital ist.

3. System zum Verfolgen der optischen Achse eines Auges mit der Synchronisation einer Lichtwelle mit einem Videosignal, das Bilder umfasst, die durch verschachtelte Einzelbilder gebildet sind, wobei das System eine Videokamera (3), die das Videosignal liefern kann, und eine Lichtquelle (4), die die Lichtwelle liefern kann, umfasst, wobei die Videokamera und die Lichtquelle durch ein Stützelement (5) getragen sind, das so ausgelegt ist, dass es auf den Kopf eines Probanden eingestellt wird, um Bilder des Auges des Probanden mit Hilfe der Videokamera zu erfassen, wobei die Lichtquelle (4) in der Nähe des Zentrums des Objektivs der Videokamera (3) angeordnet ist, um einen Purkinje-Punkt zu bestimmen, wobei das System **dadurch gekennzeichnet, dass** es ein Gehäuse (2) zum Steuern des Einschaltens der Lichtquelle mit einem Modul (9) zur Versorgung der Lichtquelle (4), einer Eingangsschnittstelle (6), die das Videosignal empfangen kann, das durch die Videokamera geliefert wird, und Verarbeitungsmitteln, die aus dem empfangenen Videosignal ein Einzelbildsynchronisationssignal durch Identifikation der geraden und ungeraden Einzelbilder, die das Videosignal bilden, extrahieren und aus dem Einzelbildsynchronisationssignal, das aus dem Videosignal extrahiert wird, ein Synchronisationssignal der geraden und ungeraden Bilder, die das Videosignal bilden, bestimmen können, umfasst, wobei die Verarbeitungsmittel außerdem dazu ausgelegt sind, ein Steuersignal für das Versorgungsmodul der Lichtquelle aus einer logischen Kombination zwischen den Einzelbildsynchronisationssignalen und den Synchronisationssignalen der Bilder zu erzeugen, das dazu geeignet ist, das Einschalten der Lichtquelle nur während der Dauer eines einzigen Einzelbildes, und zwar jedes zweiten Bildes, hervorzurufen, wobei das Steuergehäuse eine Ausgangsschnittstelle umfasst, die Mitteln (7) zur Verbindung mit einem Speichermittel entspricht, das das empfangene Videosignal aufzeichnen kann, wobei das aufgezeichnete Signal so ausgelegt ist, dass es Gegenstand einer Verarbeitung ist, bei der eine punktweise Subtraktion des Helligkeitswerts zwischen zwei aufeinander folgenden Bildern durchgeführt wird, um die Position der Darstellung der Lichtquelle in einer Szene zu lokalisieren.

4. System nach Anspruch 3, **dadurch gekennzeichnet, dass** die Eingangsschnittstelle (6) und die Ausgangschnittstelle (7) analog oder digital sind.

5. System nach Anspruch 3 oder 4, **dadurch gekennzeichnet, dass** das Speichermittel aus mindestens irgendeinem der folgenden Elemente ausgewählt ist: einer optischen Platte, einem entnehmbaren Speicherelement, einem internen Speicherelement des Gehäuses (2).

6. System nach einem der Ansprüche 3 bis 5, **dadurch gekennzeichnet, dass** die Lichtquelle (4) eine Leuchtdiode (4) ist.

7. System nach Anspruch 6, **dadurch gekennzeichnet, dass** die Leuchtdiode (4) eine Strahlung emittieren kann, die im Wesentlichen in Infrarotwellenlängen liegt.

## Claims

1. A method for tracking the optical axis of an eye, comprising the synchronization of a light wave provided by a light source (4) with a video signal captured by a video camera (3) including images made up of interlaced frames, wherein said video camera and said light source are carried by a support element (5) adapted to be adjusted to the head of a patient so as to capture images of the eye of the patient using the video camera, said light source (4) being arranged near the center of the video camera (3) lens in order to determine a Purkinje point, said method being **characterized in that** it comprises the next steps of:
reception (20) of the video signal by a housing (2) to control the ignition of the light source ;
extraction (13) by housing processing means of a frame synchronizing signal (I) by identification of the even and odd frames constituting the video signal;
determination (14) by the housing processing means of a signal for synchronizing the images (II) by detection of the even and odd images constituting the video signal, the determination of the synchronizing signal for the even and odd images constituting the video signal being obtained from the frame synchronizing signal extracted from the video signal by the housing processing means ;
generation (15) of a control signal (III) for the light source by the housing processing means from a logical combination between the signals for synchronizing the frame (I) and for synchronizing the images (II), suitable to cause the ignition of the light source only for the duration of only one frame and this, for one among two images, and
emission (21) of the control signal to a module for supplying the light source able to control the ignition and the extinction of the light source according to the control signal,
and wherein the video signal is retransmitted by the housing to a storage means to be recorded therein, said recorded signal being submitted to a processing wherein a subtraction point-to-point of the luminosity value between two successive images is carried out in order to locate, in a scene, the position of the representation of the light source.

2. The method as claimed in claim 1, wherein the video signal is an analogical or numerical one.

3. A device for tracking the optical axis of an eye, providing the synchronization of a light wave with a video signal including images made up of interlaced frames, said device comprising a video camera (3) able to provide the video signal and a light source (4) able to provide the light wave, said video camera and said light source being carried by a support element (5) adapted to be adjusted to the head of a patient so as to capture images of the eye of the patient using the video camera, said light source (4) being arranged near the center of the video camera (3) lens in order to determine a Purkinje point, said device being **characterized in that** it comprises a housing (2) to control the ignition of the light source comprising a module (9) intended to supply the light source (4), an input interface (6) able to receive the video signal provided by the video camera, and processing means intended to extract, from the received video signal, a frame synchronizing signal by identification of the even and odd frames constituting the video signal and to determine, from the frame synchronizing signal extracted from the video signal, a synchronizing signal of the even and odd images constituting the video signal, said processing means being further adapted to generate a control signal toward the light source supplying module, from a logical combination between the signals for frames synchronizing and images synchronizing, suitable to cause the ignition of the light source only for the duration of only one frame and this, for one among two images, the control housing comprising an output interface corresponding to connecting means (7) with a storage means able to record the received video signal, said recorded signal being adapted to be the submitted to a processing wherein a subtraction point-to-point of the luminosity value between two successive images is carried out in order to locate, in a scene, the position of the representation of the light source.

4. The device as claimed in claim 3, **characterized in that** said input (6) and output (7) interfaces are analogical or numerical ones.

5. The device as claimed in claim 3 or 4, **characterized in that** the storage means is selected among any one of the following elements: an optical disc, a removable storage element, an internal storage element of the housing (2).

6. The device as claimed in any of claims 3 to 5, **characterized in that** said light source (4) is an electroluminescent diode (4).

7. The device as claimed in claim 6, **characterized in that** said electroluminescent diode (4) is able to emit a radiation substantially comprised within the infra-red wavelengths.
